# NEUE EUROPÄISCHE PATENTSCHRIFT

(11) **EP 2 328 623 B2**
(45) Veröffentlichungstag und Bekanntmachung des Hinweises auf die Entscheidung über den Einspruch: **03.04.2024**
(45) Hinweis auf die Patenterteilung: 24.12.2014
(21) Anmeldenummer: 09805986.8
(22) Anmeldetag: 27.08.2009
(51) Int. Cl.: A61L 2/00, C07K 14/435, C12N 9/94

(54) **VERFAHREN ZUR VERRINGERUNG DER VIRALEN UND MIKROBIELLEN BELASTUNG FESTSTOFFHALTIGER AUS DEM PANKREAS VON TIEREN GEWONNENER EXTRAKTE**
METHOD FOR REDUCING THE VIRAL AND MICROBIAL LOAD OF EXTRACTS CONTAINING SOLIDS AND OBTAINED FROM ANIMAL PANCREAS
PROCÉDÉ POUR RÉDUIRE LA CHARGE VIRALE ET MICROBIENNE DANS DES EXTRAITS RENFERMANT DES MATIÈRES SOLIDES OBTENUS A PARTIR DE PANCREAS ANIMAL

(30) Priorität: 27.08.2008 DE 102008039860; 12.06.2009 EP 09007797; 16.06.2009 WO PCT/EP2009/004318
(43) Veröffentlichungstag der Anmeldung: 08.06.2011
(73) Patentinhaber: Nordmark Pharma GmbH, 25436 Uetersen (DE)
(72) Erfinder: RÄMSCH, Christian, 25436 Gross Nordende (DE); SCHRÄDER, Thomas, 25436 Uetersen (DE); MOEST, Thomas, 25436 Moorrege (DE); KURFÜRST, Manfred, 25436 Moorrege (DE)
(74) Vertreter: Gerbaulet, Hannes
(86) Internationale Anmeldenummer: PCT/EP2009/006216
(87) Internationale Veröffentlichungsnummer: WO 2010/022946

(56) Entgegenhaltungen:
- EP-B1- 0 115 023
- WO-A-00/48641
- WO-A-02/056824
- WO-A-2007/014896
- CA-A- 394 981
- US-A- 4 623 624
- US-A1- 2002 182 107

## Beschreibung

### Anwendungsgebiet

Die Erfindung betrifft ein Verfahren zur Verringerung der viralen und mikrobiellen Belastung feststoffhaltiger biologischer Extrakte, ein mittels dieses Verfahrens hergestelltes feststoffhaltiges biologisches Extrakt, sowie Verwendungen eines solchen feststoffhaltigen biologischen Extraktes.

### Stand der Technik

Extrakte, die aus biologischem Ausgangsmaterial gewonnen werden, können eine hohe Virenbelastung aufweisen. Viren sind Nukleinsäuren, die von einer Proteinhülle umgeben sind. Bei behüllten Viren kommt noch eine äußere Lipidhülle hinzu. Da sich Viren nicht eigenständig replizieren können, sind sie auf Wirte angewiesen. Dem entsprechend kommen sie in praktisch allen Lebewesen der Erde vor. Die wenigsten der bekannten Viren sind für den Menschen pathogen, da sie eine hohe Wirtsspezifität besitzen. Um eine Gefährdung der Konsumenten von vom herein auszuschließen, sollten Extrakte, die für den menschlichen Verzehr bestimmt sind oder die als Wirkstoff in Arzneimitteln eingesetzt werden, grundsätzlich eine möglichst geringe bzw. keine Virenbelastung aufweisen. Nicht immer führt das eigentliche Produktionsverfahren zu einer signifikanten Inaktivierung oder Entfernung der vorhandenen Viren, so dass, insbesondere bei der Herstellung pharmazeutischer Wirkstoffe, zusätzliche Abreicherungs- oder Inaktivierungsschritte in das Verfahren integriert werden müssen.

Die WO-A-2007/014896 offenbart die Wärmebehandlung eines Pankreatinextraktes zur Verringerung der viralen Belastung. US 4,623,624 A offenbart ein Verfahren zum Herstellen von Pankreatin, während US 2002/0182107 A1 ein Sterilisationsverfahren beschreibt.

Für die Abreicherung oder Inaktivierung von Viren und Mikroorganismen sind zahlreiche Methoden bekannt. Neben der mechanischen Entfernung durch z. B. Chromatographie oder Filtration können diese Kontaminationen durch Zusatz chemischer Verbindungen selektiv inaktiviert werden. Letzteres Verfahren ist aber in sofern problematisch, als diese Verbindungen wieder vollständig entfernt werden müssen, damit sie im Endprodukt keine toxischen Effekte hervorrufen. Physikalische Methoden, wie z. B. Hitzebehandlung oder Bestrahlung sind ebenfalls gängige Verfahren zur Inaktivierung von Viren oder Mikroorganismen.

Eine besondere Herausforderung ist die Inaktivierung oder Entfernung von Viren aus komplexen biologischen Extrakten, deren Wirksubstanz Enzymgemische sind, ohne dabei die enzymatische Aktivität der Proteine zu zerstören oder zu verändern.

Von besonderem wirtschaftlichen Interesse ist der pharmazeutische Wirkstoff Pankreatin, der als Extrakt aus der Schweinepankreas gewonnen wird und in getrockneter Form als orales Therapeutikum Anwendung findet, wie in DE 3248588 A1 beschrieben.

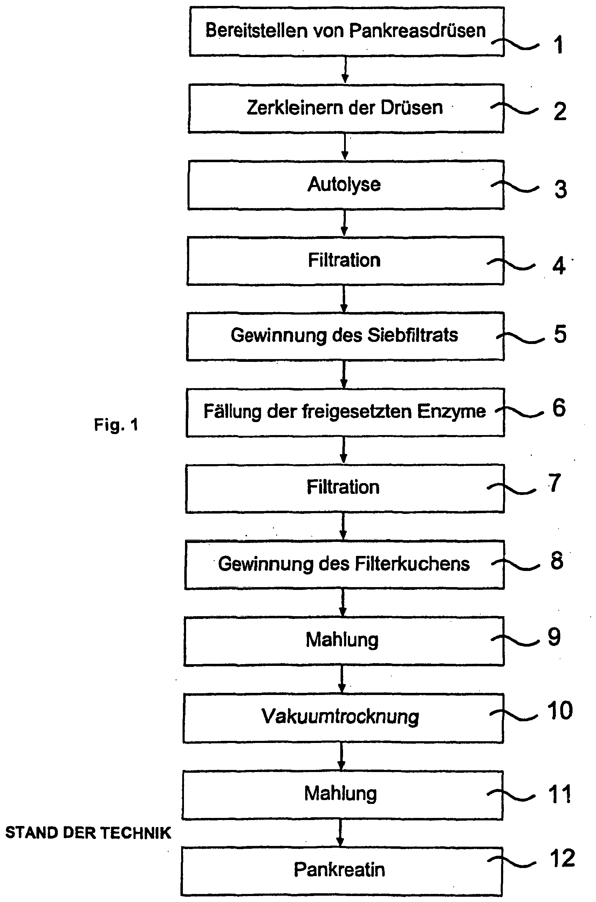

Ein typisches Verfahren zur Herstellung von Pankreatin wird nachstehend unter Bezugnahme auf Fig. 1 beschrieben. Die von Hausschweinen stammenden Pankreasdrüsen 1 werden zunächst zerkleinert 2 und einer Autolyse 3 unterzogen. Durch Filtration 4 des so erhaltenen Zwischenproduktes wird das Siebfiltrat gewonnen 5. Die Enzyme, die sich in dem Siebfiltrat befinden, werden anschließend ausgefällt 6, das Gemisch filtriert 7 und der Filterkuchen gewonnen 8. Der gewonnene Filterkuchen wird schließlich gemahlen 9, vakuumgetrocknet 10 und erneut gemahlen, wodurch das Pankreatin erhalten wird. Die mit den Bezugszeichen 2 bis 10 gekennzeichneten Verfahrensschritte führen jeweils zu Zwischenprodukten, die im Folgenden als Zwischenstufen bezeichnet werden.

Die Wirksubstanzen im Pankreatin sind u. a. verschiedene polymerabbauende Enzyme, wie Lipasen, Amylasen und Proteasen. Eine Voraussetzung für die Wirksamkeit des Therapeutikums ist, dass alle Enzyme in einem bestimmten Verhältnis und in aktiver Form im Wirkstoff vorhanden sind. Eine Besonderheit des Pankreatins ist, dass die enthaltenen Enzyme teilweise in Lösung und teilweise an Partikel gebunden vorliegen und es sich somit um eine Suspension handelt.

Untersuchungen zur Virusbelastung von Pankreatin haben gezeigt, dass behüllte, nicht aber unbehüllte Viren durch den aktuellen Produktionsprozess signifikant inaktiviert werden Grundsätzlich sollten pharmazeutische Wirkstoffe keine infektiösen Viren enthalten. Da die aktuellen Produktionsprozesse offenbar nicht in der Lage sind, eine potentiell vorhandene Kontamination von unbehüllten Viren mit ausreichender Sicherheitsmarge zu beseitigen, müssen zusätzliche virenreduzierende Schritte implementiert werden.

Klassische vireninaktivierende Methoden wie z.B. trockene oder feuchte Hitze oder virenabreichernde Methoden wie z. B. Filtration oder Chromatographie lassen sich bei Extrakten aus biologischem Ausgangsmaterial und insbesondere bei Organextrakten ohne Veränderung der Zusammensetzung und/oder hohe Produktverluste in den meisten Fällen nicht anwenden. Ein besonderes Problem dieser Extrakte sind häufig nicht gelöste Bestandteile, die ihnen eine suspensionsartige Eigenschaft verleihen. Dadurch kommt es zur Verblockung von Filtern oder Chromatographiesäulen. Weiterhin sind die wirksamen Substanzen oft sowohl in der gelösten als auch in der partikulären Fraktion verteilt und werden somit durch mechanische Abtrennverfahren teilweise entfernt.

Weitere Verfahren zur Inaktivierung von Viren, Bakterien und Pilzen in biologischen Materialien sind bekannt. Beispielsweise beschreibt WO 02/056824 A2 die Inaktivierung von Pathogenen in biologischen Materialien durch die Anwendung eines hohen Druckes. Die Erfindung bezieht sich auf die Behandlung von Blutplasma, also einer Lösung von biologischen aktiven Stoffen in Wasser. Bradley D. W. et al. beschreiben in "Pressure cycling technology: a novel approach to virus inactivation in plasma" (Transfusion, 40, 2000, 193) ebenfalls ein Verfahren zur Behandlung von Blutplasma.

Die Hochdruck-Behandlung von Lebensmitteln ist verschiedentlich beschrieben worden. Beispielsweise können Muscheln mit hohem Druck behandelt werden, um Noroviren oder Hepatitis-A-Viren zu inaktivieren (Kingsley et al., Inactivation of a Norovirus by High-Pressure Processing, Appl. Environ. Microbiol. 2007, 581; Calci et al., High-Pressure Inactivation of Hepatitis A Virus within Oysters, Appl. Environ. Microbiol. 2005, 339). Die Behandlung soll die organoleptischen Eigenschaften des Lebensmittels erhalten. Die biologische Aktivität von Enzymen oder anderen Wirkstoffen nach der Hochdruckbehandlung wurde nicht untersucht. Überdies wurden in beiden Fällen Feststoffe untersucht.

Es ist jedoch auch bekannt, dass nicht ohne weiteres vorhergesagt werden kann, ob mittels Hochdruckbehandlung tatsächlich eine Inaktivierung bestimmter Viren gelingt (Grove S. F. et al., Inactivation of Foodborne Viruses of Significance by High Pressure and other Processes, J. Food Prot. 2006, 667). Je nach dem, ob es sich um flüssige Proben oder feste Proben handelt, müssen aufgrund der unterschiedlichen Kompressibilität der Proben unterschiedliche Verfahrensbedingungen gewählt werden.

Außerdem kann die Hochdruckbehandlung zu einer Veränderung der Lebensmittel führen. Beispielsweise werden Früchte braun, wenn sie einer solchen Behandlung unterzogen werden. Diese Farbänderung ist auf eine enzymatische Reaktion zurückzuführen, was auf eine Veränderung der biologischen Aktivität dieser Enzyme schließen lässt.

Eine besondere Schwierigkeit besteht bei biologischen Proben, die nicht in weitgehend homogener Form vorliegen. Bei einem feststoffhaltigen Extrakt in Form einer Suspension befinden sich Teile der biologisch aktiven Wirkstoffe in der flüssigen Phase, andere Teile in den dispergierten Feststoffteilchen. Eine Inaktivierung von Viren in den Feststoffteilen kann daher mit einer Zerstörung der Wirkstoffe verbunden sein, die sich in der flüssigen Phase befinden, da die Kompressibilität der flüssigen Phase höher als die der Feststoffteilchen ist.

Pankreatin oder die bei der Herstellung anfallenden Zwischenstufen stellen aufgrund ihrer natürlichen Virusbelastung und ihres Suspensionscharakters beispielhafte feststoffhaltige biologische Extrakte dar. Bei klassischen Spiking-Experimenten wird die entsprechende Substanz mit verschiedenen Laborstämmen gespikt und der Titer vor und nach der Behandlung bestimmt.

Es besteht darüber hinaus ein Bedarf an Verfahren, bei denen die in einem feststoffhaltigen biologischen Extrakt enthaltenen viralen und bakteriellen Belastungen vollständig oder auf ein Minimum reduziert werden. Das Verfahren muss gleichermaßen für Feststoffe und Suspensionen geeignet sein. Das Verfahren soll insbesondere die Inaktivierung von unbehüllten Viren im Pankreatin ermöglichen.

### Aufgabe, Lösung, Vorteil

Aufgabe der Erfindung ist es, die Nachteile nach dem Stand der Technik zu beseitigen. Es soll insbesondere ein Verfahren zur Verringerung der viralen und mikrobiellen Belastung feststoffhaltiger biologischer Extrakte angegeben werden, das für Feststoffe und Suspensionen geeignet ist, die Aktivität der in dem biologischen Extrakt enthaltenen Enzyme nicht wesentlich verringert, die pharmakologisch gewünschten Eigenschaften nicht verschlechtert und keine toxischen chemischen Verbindungen erzeugt.

Ferner sollen mittels des Verfahrens hergestellte Extrakte und Verwendungen derartiger Extrakte angegeben werden.

Diese Aufgabe wird durch die Merkmale von Anspruch 1 gelöst. Zweckmäßige Ausgestaltungen der Erfindung ergeben sich aus den Merkmalen der Unteransprüche.

Nach Maßgabe der Erfindung ist ein Verfahren zur Verringerung der viralen und mikrobiellen Belastung feststoffhaltiger biologischer Extrakte vorgesehen, umfassend die Schritte
(a) Bereitstellen des feststoffhaltigen biologischen Extraktes, umfassend zumindest einen biologisch aktiven Wirkstoff, ausgewählt aus Enzymen, Proteinen und Peptiden, oder ein Gemisch derartiger Wirkstoffe; und
(b) Unterziehen des in Schritt (a) bereitgestellten Extraktes einer Hochdruckbehandlung bei einem Druck im Bereich von 3000 bis 6000 bar;

wobei die biologische Aktivität des feststoffhaltigen biologischen Extraktes nach der Hochdruckbehandlung zumindest 50 % der biologischen Aktivität des feststoffhaltigen biologischen Extraktes vor der Hochdruckbehandlung entspricht, und die virale Infektiosität des feststoffhaltigen biologischen Extraktes nach der Hochdruckbehandlung um einen Faktor von größer als 1 log10 im Vergleich zur viralen Infektiosität vor der Hochdruckbehandlung verringert worden ist,
dadurch gekennzeichnet,
dass der der feststoffhaltige biologische Extrakt aus dem Pankreas von Tieren gewonnen wurde und
dass der feststoffhaltige biologische Extrakt als Suspension, umfassend eine flüssige Phase und darin dispergierte Feststoffteilchen, bereitgestellt wird, wobei das Gemisch biologisch aktiver Wirkstoffe zu einem Teil in der flüssigen Phase gelöst und zu einem anderen Teil an die Feststoffteilchen gebunden ist,
wobei der feststoffhaltige biologische Extrakt vor der Hochdruckbehandlung einem Filtrationsschritt unterzogen wird, wodurch ein Filtrat und ein Filterkuchen erhalten werden, wobei der Filterkuchen der Hochdruckbehandlung unterzogen wird und
wobei der Filterkuchen einen Feststoffanteil von zumindest 40 Gew.-%, bezogen auf das Gewicht des Filterkuchens, aufweist.

Unter dem Begriff "feststoffhaltiger biologischer Extrakt" soll hier ein Extrakt verstanden werden, der einen biologisch aktiven Wirkstoff, ausgewählt aus Enzymen, Proteinen und Peptiden, oder ein Gemisch derartiger Wirkstoffe umfasst. Es ist bevorzugt, dass der feststoffhaltige biologische Extrakt ein Gemisch der genannten biologisch aktiven Wirkstoffe umfasst. Im Folgenden wird der feststoffhaltige biologische Extrakt der Einfachheit halber auch als Extrakt bezeichnet.

Der feststoffhaltige biologische Extrakt ist vorzugsweise ein Extrakt, der als alkoholischer oder wässeriger Extrakt aus tierischen Organen gewonnen wurde. Der enthaltene biologisch aktive Wirkstoff oder das Wirkstoffgemisch können in dem Extrakt sowohl in Lösung als auch an Feststoffe gebunden vorliegen. Ein solches feststoffhaltiges biologisches Extrakt ist ein komplexer Extrakt. Der biologisch aktive Wirkstoff oder das Gemisch derartiger Wirkstoffe weist vorzugsweise pharmazeutische Aktivität auf.

Unter dem Begriff "biologisch aktiver Wirkstoff" werden pharmazeutische Wirkstoffe und therapeutische Wirkstoffe verstanden. Biologisch aktive Wirkstoffe im Sinne dieser Erfindung sind beispielsweise Enzyme, Proteine und Peptide.

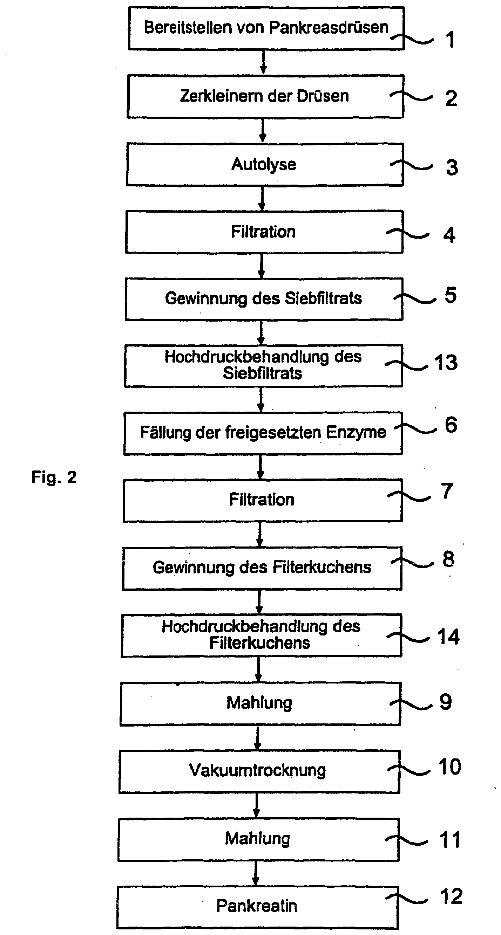

Beispiele feststoffhaltiger biologischer Extrakte sind Extrakte, die insbesondere aus dem Pankreas des Schweins, gewonnen werden. Spezielle Beispiele sind das Pankreatin sowie die bei dessen Herstellung anfallenden Zwischenstufen. Diese Zwischenstufen sind in Fig. 2 mit den Bezugszeichen 2 bis 10 gekennzeichnet. Bevorzugte Beispiele dieser Zwischenstufen sind das Siebfiltrat (Bezugszeichen 5 in Fig. 2) und ein Beispiel des Filterkuchens (Bezugszeichen 8 in Fig. 2), nach deren Gewinnung eine Hochdruckbehandlung durchgeführt wird, d. h. der Filterkuchen und vorzugsweise zusätzlich das Siebfiltrat können einer Hochdruckbehandlung unterzogen werden (Bezugszeichen 13 bzw. 14 in Fig. 2). Aus dem Pankreas wird Pankreatin als pharmazeutischer Wirkstoff gewonnen. Pankreatin sowie die bei der Herstellung anfallenden Zwischenstufen enthalten u. a. die Enzyme Lipase, Amylase und Protease. Sie sind somit feststoffhaltige biologische Extrakte im Sinne der vorliegenden Erfindung. Die Extrakte werden vorzugsweise als wässerig-alkoholische Extrakte in Schritt (a) des erfindungsgemäßen Verfahrens bereitgestellt.

Das erfindungsgemäße Verfahren ist auf alle Virusformen, wie DNA- und RNA-Viren, behüllte und unbehüllte Viren, weiterhin auf Virionen und Prionen oder ähnliche biologische Systeme sowie Bakterien und Pilze anwendbar. Das Verfahren wird bevorzugt zur Verringerung der Belastung von unbehüllten Viren im Pankreatin aus dem Schweine-Pankreas oder in entsprechenden bei der Herstellung anfallenden Zwischenstufen (siehe Fig. 2) verwendet.

Das erfindungsgemäße Verfahren erlaubt die Reduktion der Viren- und Mikroorganismen-Belastung von feststoffhaltigen biologischen Extrakten, ohne dass sich deren enzymatische Aktivität wesentlich verringert, die pharmakologisch beabsichtigten Eigenschaften verschlechtern oder toxische chemische Verbindungen erzeugt werden.

Die biologische Aktivität des feststoffhaltigen biologischen Extraktes nach der Hochdruckbehandlung sollte zumindest 50 % der biologischen Aktivität des feststoffhaltigen biologischen Extraktes vor der Hochdruckbehandlung betragen, bevorzugt zumindest 80 %, besonders bevorzugt zumindest 90 %.

Ist zumindest einer der biologisch aktiven Wirkstoffe, die in dem Extrakt enthalten sind, ein Enzym, so sollte die enzymatische Aktivität des feststoffhaltigen biologischen Extraktes nach der Hochdruckbehandlung zumindest 50 % der enzymatischen Aktivität des feststoffhaltigen biologischen Extraktes vor der Hochdruckbehandlung betragen, bevorzugt zumindest 80 %, besonders bevorzugt zumindest 90 %.

Die virale Infektiosität des feststoffhaltigen biologischen Extraktes nach der Hochdruckbehandlung ist um einen Faktor von größer als 1 log10, vorzugsweise größer 3 log10, besonders bevorzugt größer 4 log10, im Vergleich zur viralen Infektiosität vor der Hochdruckbehandlung verringert worden. Dies gilt insbesondere auch für die virale Infektiosität von unbehüllten Viren. Beispielsweise sollte die Infektiosität von unbehüllten Viren im Pankreatin nach der Behandlung um einen Faktor von größer als 1 log1o, vorzugsweise größer 3 log1o. besonders bevorzugt größer 4 log10 im Vergleich zu dessen Infektiosität vor der Behandlung verringert sein. Vorzugsweise beträgt die Keimbelastung nach der Hochdruckbehandlung nicht mehr als 500 KBE/g, bevorzugt nicht mehr als 100 KBE/g.

Bei der Hochdruckbehandlung des Extraktes werden keine chemischen Substanzen zugesetzt, so dass die Belastung mit toxischen Substanzen nach der Behandlung nicht höher als vor der Behandlung ist.

Die Hochdruckbehandlung erfolgt vorzugsweise bei konstantem Druck oder durch Druckimpulse und kann im Durchflussverfahren oder im Batchverfahren in entsprechenden Apparaturen durchgeführt werden. Die Einwirkzeit der Hochdruckbehandlung auf das in Schritt (a) bereitgestellte Extrakt beträgt vorzugsweise 1 bis 60 min. Die Hochdruckbehandlung wird bevorzugt bei Temperaturen zwischen -20 °C und 30 °C, besonders bevorzugt zwischen -10 °C und 20 °C durchgeführt.

Die flüssige Phase des Extraktes, der einer Hochdruckbehandlung in Schritt (b) unterzogen wird, kann als Lösungsmittel Wasser oder ein Gemisch aus Wasser und einem organischen Lösungsmittel enthalten. Das verwendete Lösungsmittel ist vorzugsweise ein Alkohol, besonders bevorzugt Isopropanol.

Ein feststoffhaltiger biologischer Extrakt, der durch das erfindungsgemäße Verfahren erhalten wurde ist durch geringe virale und mikrobielle Belastung gekennzeichnet. Trotz der vorherigen Hochdruckbehandlung ist seine biologische, insbesondere seine enzymatische Aktivität nicht wesentlich verringert, sind seine pharmakologisch beabsichtigen Eigenschaften nicht verschlechtert, und er weist keine toxischen chemischen Verbindungen auf, die bei der Behandlung zugegeben wurden.

Der erfindungsgemäße, durch Hochdruckbehandlung erhaltene Extrakt bzw. der daraus hergestellte biologische Wirkstoff kann für die Herstellung von Arzneimitteln, insbesondere im Rahmen der oralen Enzymtherapie, sowie als Nahrungs- oder Lebensmittel oder Nahrungsergänzungsmittel verwendet werden.

### Kurzbeschreibung der Zeichnung

Die Erfindung wird nachfolgend anhand von Beispielen, die die Erfindung nicht beschränken sollen, unter Bezugnahme auf die Zeichnungen näher erläutert. Dabei zeigen
Fig. 1 ein Ablaufschema eines Verfahrens zur Herstellung von Pankreatin aus Schweine-Pankreasdrüsen nach dem Stand der Technik;
Fig. 2 ein Ablaufschema eines Verfahrens zur Herstellung von Pankreatin aus Schweine-Pankreasdrüsen;
Fig. 3 ein Diagramm, das die relativen enzymatischen Aktivitäten nach Behandlung von Siebfiltrat aus dem Pankreatin-Produktionsprozess mit unterschiedlichen Drücken in Prozent, bezogen auf die Ausgangsaktivität der unbehandelten Probe, zeigt;
Fig. 4 ein Diagramm, das die relativen enzymatischen Aktivitäten nach Behandlung von Filterkuchen aus dem Pankreatin-Produktionsprozess mit unterschiedlichen Drücken in Prozent, bezogen auf die Ausgangsaktivität der unbehandelten Probe, zeigt.
Fig. 5, Fig. 6

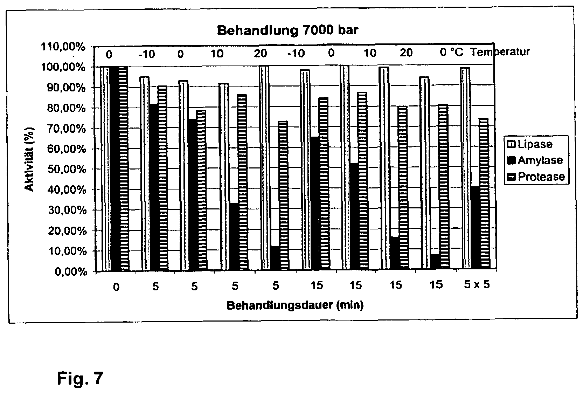

   und Fig. 7 Diagramme, die die relativen enzymatischen Aktivitäten nach Behandlung von Filterkuchen aus dem Pankreatin-Produktionsprozess mit unterschiedlichen Drücken und unterschiedlichen Temperaturen in Prozent, bezogen auf die Ausgangsaktivität der unbehandelten Probe, zeigen.

### Detaillierte Beschreibung der Erfindung und bester Weg zur Ausführung der Erfindung

Eine Ausführungsform eines Verfahrens zur Herstellung von Pankreatin wird nachstehend unter Bezugnahme auf Fig. 2 beschrieben. Die von Hausschweinen stammenden Pankreasdrüsen 1 werden zunächst zerkleinert 2 und einer Autolyse 3 unterzogen. Durch Filtration 4 des so erhaltenen Zwischenproduktes wird das Siebfiltrat gewonnen 5. Das Siebfiltrat kann vor der Weiterbehandlung einer Hochdruckbehandlung unterzogen werden 13. Anschließend werden die Enzyme, die
sich in dem Siebfiltrat befinden, ausgefällt 6, das Gemisch filtriert 7 und der Filterkuchen gewonnen 8. Der gewonnene Filterkuchen kann einer Hochdruckbehandlung unterzogen werden 14. Der Filterkuchen wird schließlich gemahlen 9, vakuumgetrocknet 10 und erneut gemahlen, wodurch das Pankreatin erhalten wird 12.

Gemäß dem in Fig. 2 dargestellten Verfahren kann (i) eine Hochdruckbehandlung des Siebfiltrates 13 oder (ii) eine Hochdruckbehandlung des Filterkuchens 14 oder (iii) eine Hochdruckbehandlung des Siebfiltrates 13 und eine Hochdruckbehandlung des Filterkuchens 14 vorgesehen sein.

### Beispiel 1

Das folgende Beispiel beschreibt die Behandlung von Zwischenstufen aus dem Pankreatin-Herstellungsverfahren als feststoffhaltige biologische Extrakte, die aus dem Schweine-Pankreas gewonnen wurden, mit Hochdruck.

### (1) Durchführung

Die Behandlung wurde im Batchverfahren in einer Hochdruckapparatur durchgeführt.
a) Eine in 40%igem Isopropanol gelöste Zwischenstufe des Pankreatin-Produktionsprozesses (Siebfiltrat mit ca. 5 Gew.-% Feststoffanteil, bezogen auf das Gewicht des Siebfiltrats, siehe Bezugszeichen 13 von Fig. 2) wurde für 5 min bei 15 °C einer Hochdruckbehandlung bei 4000, 5000 und 6000 bar unterzogen.
b) Alternativ wurde unter den gleichen Bedingungen eine Hochdruckbehandlung mit Filterkuchen (siehe Bezugszeichen 14 von Fig. 2, Feststoffanteil ca. 50 Gew.-%, Flüssiganteil, bestehend zu ca. 80 Gew.-% aus Isopropanol und zu ca. 20 Gew.-% aus Wasser) durchgeführt.

Für beide Proben wurde die Aktivität von Lipase, Amylase und Protease im Vergleich zu einer unbehandelten Probe bestimmt.

### (2) Bewertung

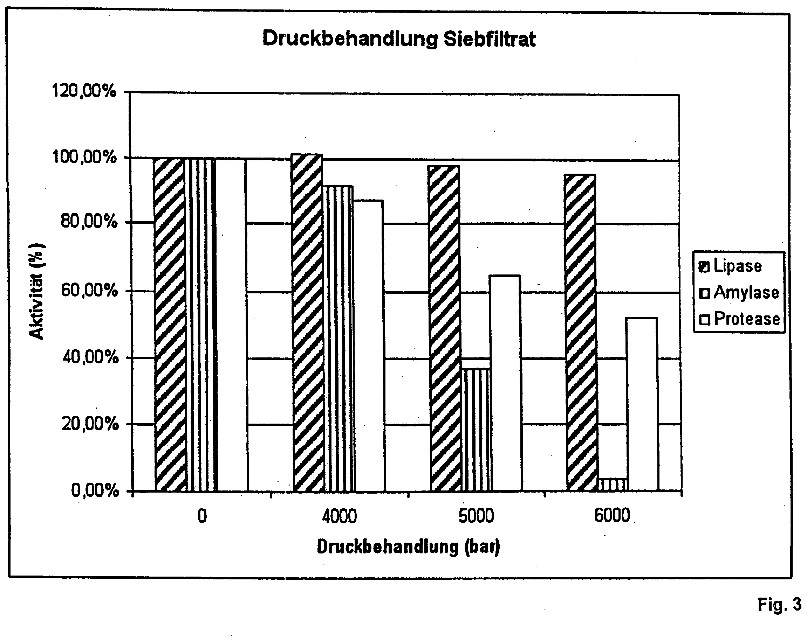

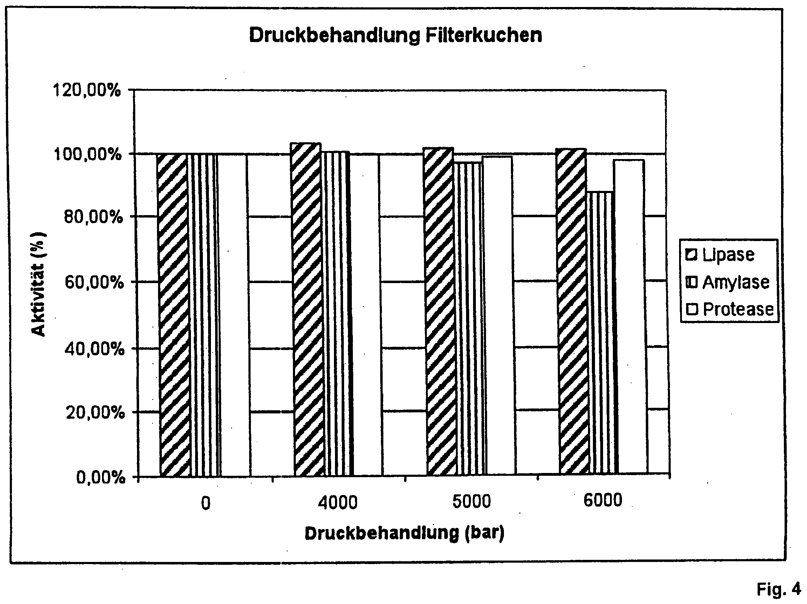

Die dargestellten Ergebnisse zeigen, dass die drei gemessenen Enzymaktivitäten im Filterkuchen (Fig. 2) auch bei einer Hochdruckbehandlung mit 6000 bar nicht signifikant abnehmen (Fig. 4). Die Lipaseaktivität war auch im Siebfiltrat (Fig. 2) bei allen Drücken stabil. Im Gegensatz dazu nahm die Amylaseaktivität im Siebfiltrat bei Drücken > 4000 bar deutlich ab (Fig. 3).

### Beispiel 2

Das folgende Beispiel beschreibt die Behandlung von Filterkuchen aus dem Pankreatin-Herstellungsverfahren, der aus dem Schweine-Pankreas gewonnen wurde, mit Hochdruck.

### (1) Durchführung

Die Behandlung wurde im Batchverfahren in einer Hochdruckapparatur durchgeführt.

Filterkuchen (siehe Bezugszeichen 14 von Fig. 2, Feststoffanteil ca. 50 Gew.-%, Flüssiganteil, bestehend zu ca. 80 Gew.-% aus Isopropanol und zu ca. 20 Gew.-% aus Wasser) wurde bei verschiedenen Drücken und Temperaturen einer Hochdruckbehandlung unterzogen.

Für alle Proben wurde die Aktivität von Lipase, Amylase und Protease im Vergleich zu einer unbehandelten Probe bestimmt.

### (2) Bewertung

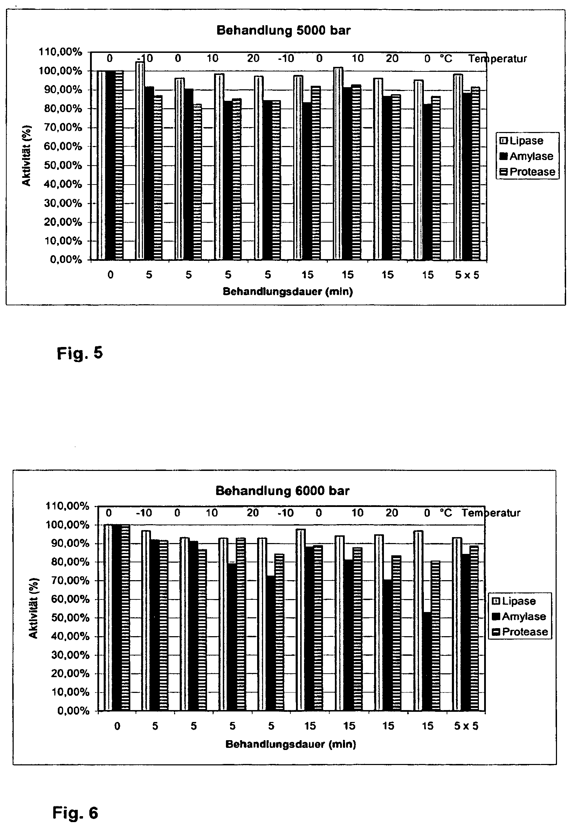

Die dargestellten Ergebnisse zeigen, dass die Inaktivierung der Enzyme im Filterkuchen (Fig. 2) sowohl vom Druck als auch von der Temperatur abhängig ist. Die Lipaseaktivität wies die höchste Stabilität auf und wurde auch bei Drücken von 7000 bar nicht signifikant inaktiviert (Fig. 5). Im Gegensatz dazu zeigte die Amylase die größte Empfindlichkeit gegenüber einer Hochdruckbehandlung. Niedrige Temperaturen hatten einen stabilisierenden Einfluss auf die Enzymaktivitäten (Fig. 5).

### Beispiel 3

Das folgende Beispiel beschreibt die Inaktivierung des unbehüllten Virus FCV mittels Hochdruck (FCV = Felines Calicivirus ).

### (1) Durchführung

Die Behandlung wurde im Batchverfahren in einer Hochdruckapparatur durchgeführt.

Eine FCV-Stocklösung in Kulturmedium wurde bei unterschiedlichen Drücken und Temperaturen für unterschiedliche Zeiten einer Hochdruckbehandlung unterzogen. Der Virustiter wurde in der Ausgangsprobe und in den behandelten Proben bestimmt.

### (2) Bewertung

Die dargestellten Ergebnisse (Tab. 1) zeigen, dass der Virustiter durch die Hochdruckbehandlung bei allen Proben, außer bei der Probe, die bei 20 °C, 5 min mit 4000 bar behandelt worden war, bis unter die Nachweisgrenze von 1,9 log sinkt. Aus der Differenz der Virustiter zwischen unbehandelter Probe und behandelten Proben ergibt sich ein Abreicherungsfaktor von ^{~} 6,3 log. FCV wird als Modellvirus für den ebenfalls unbehüllten HEV verwendet, der bei Schweinen nachgewiesen wurde und als human pathogen gilt, aber selber nicht in Zellkultur nachgewiesen werden kann. Eine Abreicherung dieses Virus um > 6 log Stufen, stellt eine wesentliche Verbesserung der biologischen Sicherheit von Pankreatin dar.

**Tabelle 1; Virustiter nach Hochdruckbehandlung einer FCV Lösung**

| **Behandlung** | | **4000 bar** | **5000 bar** | **6000 bar** |
|---|---|---|---|---|
| **Zeit (min)** | **Temperatur (°C)** | | | |
| 0 | 20 | 8,2 log | | |
| 5 | 20 | 3,6 log | < 1,9 log | < 1,9 log |
| 5 | 20 | < 1,9 log | < 1,9 log | < 1,9 log |
| 5 | 20 | < 1,9 log | < 1,9 log | < 1,9 log |
| 5 | 0 | < 1,9 log | < 1,9 log | < 1,9 log |
| 5 | 0 | < 1,9 log | < 1,9 log | < 1,9 log |
| 5 | 0 | < 1,9 log | < 1,9 log | < 1,9 log |
| 5 | -5 | < 1,9 log | < 1,9 log | < 1,9 log |
| 5 | -5 | < 1,9 log | < 1,9 log | < 1,9 log |
| 5 | -5 | < 1,9 log | < 1,9 log | < 1,9 log |
| 15 | 0 | < 1,9 log | < 1,9 log | < 1,9 log |
| 15 | 0 | < 1,9 log | < 1,9 log | < 1,9 log |
| 15 | 0 | < 1,9 log | < 1,9 log | < 1,9 log |

### Beispiel 4

Das folgende Beispiel beschreibt die Inaktivierung der unbehüllten Viren FCV, EMCV (Encephalomyocarditis Virus) und Reo3 mittels Hochdruck.

### (1) Durchführung

Die Behandlung wurde im Batchverfahren in einer Hochdruckapparatur durchgeführt.

Die drei Viren· wurden in den Filterkuchen des Pankreatin-Produktionsprozesses gespikt und bei unterschiedlichen Temperaturen und für unterschiedliche Zeiten bei 6000 bar einer Hochdruckbehandlung unterzogen. Der Virustiter wurde in den Ausgangsproben (Load-Proben), in den behandelten Proben und in den unbehandelten Proben nach Durchführung der Hochdruckbehandlung (Hold-Proben} bestimmt.

### (2) Bewertung

Die dargestellten Ergebnisse (Tab. 2) zeigen, dass der Titer aller untersuchten Viren durch die Hochdruckbehandlung signifikant reduziert wurde. Für FCV ergibt sich ein Reduktionsfaktor von 3,82 log. Die beiden anderen Viren wurden bis auf einen Titer unterhalb der Nachweisgrenze von 1,5 log reduziert. Aus den entsprechenden Load-Titern ergeben sich Reduktionsfaktoren von^{~} 4,98 log für EMCV und^{~} 3,78 log für Reo3. Eine Reduktion der Titer dieser drei zoonotischen Viren (FCV = Modell für HEV) um mehr als 3,5 log Stufen, stellt eine wesentliche Verbesserung der biologischen Sicherheit von Pankreatin dar. Mit diesem Versuch konnte gezeigt werden, dass der Titer verschiedener Viren in Prozessintermediaten des Pankreatin-Produktionsprozesses durch eine Hochdruckbehandlung signifikant reduziert werden kann. Eine optimale Virusinaktivierung wird unter den Bedingungen erreicht, die gleichzeitig eine maximale Stabilität der im Pankreatin vorhandenen Enzyme gewährleisten (Temperaturen^{~} O°C).

**Tabelle 2: Virustiter nach Hochdruckbehandlung von FCV, EMCV und Reo3 im Pankreatin Filterkuchen.**

| Behandlung | | Titer (log TCID₅₀) | | |
|---|---|---|---|---|
| Temperatur (°C) | Dauer (min) | FCV | EMCV | Reo3 |
| Load-Probe | | 6,97 | 6,48 | 5,28 |
| 0 | 5 | 4,76 | 2,97 | 2,97 |
| 0 | 15 | 5,77 | < 1,5 | 2,88 |
| -10 | 5 | 4,35 | < 1,5 | < 1,5 |
| - 10 | 15 | 3,15 | < 1,5 | < 1,5 |
| Hold-Probe | | 6,79 | 6,73 | 5,05 |

Die Ausführungsbeispiele sind ebenfalls Gegenstand der Erfindung.

## Patentansprüche

1. Verfahren zur Verringerung der viralen und mikrobiellen Belastung feststoffhaltiger biologischer Extrakte, umfassend die Schritte
(a) Bereitstellen des feststoffhaltigen biologischen Extraktes, umfassend zumindest einen biologisch aktiven Wirkstoff, ausgewählt aus Enzymen, Proteinen und Peptiden, oder ein Gemisch derartiger Wirkstoffe; und
(b) Unterziehen des in Schritt (a) bereitgestellten Extraktes einer Hochdruckbehandlung bei einem Druck im Bereich von 3000 bis 6000 bar;
wobei die biologische Aktivität des feststoffhaltigen biologischen Extraktes nach der Hochdruckbehandlung zumindest 50 % der biologischen Aktivität des feststoffhaltigen biologischen Extraktes vor der Hochdruckbehandlung entspricht und
die virale Infektiosität des feststoffhaltigen biologischen Extraktes nach der Hochdruckbehandlung um einen Faktor von größer als 1 logio im Vergleich zur viralen Infektiosität vor der Hochdruckbehandlung verringert worden ist und
**dadurch gekennzeichnet,**
**dass** der feststoffhaltige biologische Extrakt aus dem Pankreas von Tieren gewonnen wurde und
**dass** der feststoffhaltige biologische Extrakt als Suspension, umfassend eine flüssige Phase und darin dispergierte Feststoffteilchen, bereitgestellt wird, wobei das Gemisch biologisch aktiver Wirkstoffe zu einem Teil in der flüssigen Phase gelöst und zu einem anderen Teil an die Feststoffteilchen gebunden ist und
**dass** der feststoffhaltige biologische Extrakt vor der Hochdruckbehandlung einem Filtrationsschritt unterzogen wird, wodurch ein Filtrat und ein Filterkuchen erhalten werden, wobei der Filterkuchen der Hochdruckbehandlung unterzogen wird und
**dass** der Filterkuchen einen Feststoffanteil von zumindest 40 Gew.-%, bezogen auf das Gewicht des Filterkuchens, aufweist.

2. Verfahren nach Anspruch 1,
**dadurch gekennzeichnet,**
**dass** der feststoffhaltige Extrakt ein Extrakt aus dem Pankreas des Schweins ist.

3. Verfahren nach einem der vorstehenden Ansprüche,
**dadurch gekennzeichnet,**
**dass** die Hochdruckbehandlung bei einem konstanten Druck oder mit pulsierenden Drücken durchgeführt wird.

4. Verfahren nach einem der vorangehenden Ansprüche zur Herstellung von Pankreatin, umfassend die Schritte:
(i) Zerkleinern von Pankreasdrüsen von Hausschweinen;
(ii) die zerkleinerten Pankreasdrüsen werden anschließend einer Autolyse unterzogen;
(iii) das so erhaltene Zwischenprodukt wird gesiebt und das Siebfiltrat wird gewonnen;
(iv) Ausfällung der Enzyme, die sich in dem Siebfiltrat befinden;
(v) das Gemisch wird filtriert und der Filterkuchen wird gewonnen;
(vi) der Filterkuchen wird schließlich gemahlen;
(vii) nach dem Mahlen vakuumgetrocknet und
(viii) erneut gemahlen, wodurch Pankreatin erhalten wird,
**dadurch gekennzeichnet,**
**dass** der in Schritt (v) gewonnene Filterkuchen der Hochdruckbehandlung unterzogen wird.

5. Verfahren nach einem der vorstehenden Ansprüche, worin die Einwirkzeit der Hochdruckbehandlung 1 bis 60 min beträgt.

6. Verfahren nach einem der vorstehenden Ansprüche, worin die Hochdruckbehandlung bei Temperaturen im Bereich von -20 °C bis 30 °C durchgeführt wird.

7. Verfahren nach einem der vorstehenden Ansprüche, worin die Hochdruckbehandlung bei Temperaturen im Bereich von -10 °C bis 20 °C durchgeführt wird.

8. Verfahren nach einem der vorstehenden Ansprüche,
**dadurch gekennzeichnet,**
**dass** die biologische Aktivität des feststoffhaltigen biologischen Extraktes nach der Hochdruckbehandlung zumindest 90 % der biologischen Aktivität des feststoffhaltigen biologischen Extraktes vor der Hochdruckbehandlung entspricht.

9. Verfahren nach einem der vorstehenden Ansprüche, **dadurch gekennzeichnet, dass** bei der Hochdruckbehandlung des feststoffhaltigen biologischen Extraktes keine chemischen Substanzen zugesetzt werden.

## Claims

1. A method for reducing the viral and microbial contamination of solid-containing biological extracts, comprising the steps
(a) providing the solid-containing biological extract, comprising at least one biologically active agent, selected from enzymes, proteins and peptides, or a mixture of active agents of this type; and
(b) subjecting the extract provided in step (a) to a high-pressure treatment at a pressure in the range from 3000 to 6000 bar;
wherein the biological activity of the solid-containing biological extract, after the high-pressure treatment, corresponds to at least 50 % of the biological activity of the solid-containing biological extract before the high-pressure treatment, and
the viral infectivity of the solid-containing biological extract after the high pressure treatment has been reduced by a factor of greater than 1 log₁₀ compared to the viral infectivity before the high pressure treatment, and
**characterised in that**
the solid-containing biological extract was obtained from the pancreas of animals, and
**in that** the solid-containing biological extract is provided as a suspension, comprising a liquid phase and solid particles dispersed therein, wherein the mixture of biologically active agents is partly dissolved in the liquid phase and, in another part, bound to the solid particles, and
**in that** the solid-containing biological extract is subjected to a filtration step prior to the high-pressure treatment, whereby a filtrate and a filter cake are obtained, wherein the filter cake is subjected to the high-pressure treatment, and
**in that** the filter cake has a solids content of at least 40% by weight, based on the weight of the filter cake.

2. The method according to claim 1,
**characterised in that**
the solid-containing extract is an extract from porcine pancreas.

3. The method according to one of the preceding claims,
**characterised in that**
the high-pressure treatment is carried out at a constant pressure or with pulsing pressures.

4. The method according to one of the preceding claims for producing pancreatin, comprising the steps:
(i) comminuting pancreatic glands from domestic pigs;
(ii) the comminuted pancreatic glands are subsequently subjected to autolysis;
(iii) the thus-obtained intermediate product is sieved and the sieve filtrate is obtained;
(iv) precipitating the enzymes that are in the sieve filtrate;
(v) the mixture is filtered and the filter cake is obtained;
(vi) the filter cake is finally ground;
(vii) vacuum-dried after grinding, and
(viii) ground again, as a result of which pancreatin is obtained,
**characterised in that**
the filter cake obtained in step (v) is subjected to the high-pressure treatment.

5. The method according to one of the preceding claims, wherein the exposure time of the high-pressure treatment is 1 to 60 min.

6. The method according to one of the preceding claims, wherein the high-pressure treatment is carried out at temperatures in the range from -20 °C to 30 °C.

7. The method according to one of the preceding claims, wherein the high-pressure treatment is carried out at temperatures in the range from -10 °C to 20 °C.

8. The method according to one of the preceding claims,
**characterised in that**
the biological activity of the solid-containing biological extract after the high-pressure treatment corresponds to at least 90 % of the biological activity of the solid-containing biological extract before the high-pressure treatment.

9. The method according to one of the preceding claims, **characterised in that** no chemical substances are added during the high-pressure treatment of the solid-containing biological extract.

## Revendications

1. Procédé visant à réduire la contamination virale et microbienne d'extraits biologiques contenant des matières solides, lequel comprend les étapes consistant à
(a) fournir l'extrait biologique contenant des matières solides et comprenant au moins un principe biologiquement actif choisi parmi les enzymes, les protéines et les peptides, ou un mélange de tels principes actifs ; et
(b) soumettre l'extrait fourni à l'étape (a) à un traitement haute pression, réalisé à une pression comprise entre 3000 et 6 000 bar ;
l'activité biologique de l'extrait biologique contenant des matières solides correspondant, après ledit traitement haute pression, à au moins 50 % de l'activité biologique que l'extrait biologique contenant des matières solides présentait avant ce traitement haute pression, et
l'infectiosité virale de l'extrait biologique contenant des matières solides ayant été réduite, après ledit traitement haute pression, d'un facteur supérieur à 1 log₁₀, en comparaison avec l'infectiosité virale avant ce traitement haute pression, et
**caractérisé en ce que**
l'extrait biologique contenant des matières solides a été obtenu à partir de pancréas d'animaux, et
l'extrait biologique contenant des matières solides est fourni sous forme d'une suspension comprenant une phase liquide et des particules de matière solide mises en dispersion dans cette dernière, une partie du mélange de principes biologiquement actifs étant dissoute dans la phase liquide et l'autre partie de celui-ci étant liée aux particules de matière solide, et
l'extrait biologique contenant des matières solides est soumis, avant ledit traitement haute pression, à une étape de filtration permettant d'obtenir un filtrat et un gâteau de filtration, le gâteau de filtration étant soumis à ce traitement haute pression, et
le gâteau de filtration présente un taux de matières solides supérieur ou égal à 40 % en poids, par rapport au poids du gâteau de filtrage.

2. Procédé selon la revendication 1,
**caractérisé en ce que**
l'extrait contenant des matières solides est un extrait réalisé à partir de pancréas porcin.

3. Procédé selon l'une des revendications précédentes,
**caractérisé en ce que**
ledit traitement haute pression est mis en oeuvre à pression constante ou en appliquant des pressions pulsées.

4. Procédé selon l'une des revendications précédentes, visant à fabriquer de la pancréatine, lequel comprend les étapes consistant à :
(i) broyer les glandes pancréatiques de cochons domestiques ;
(ii) soumettre les glandes pancréatiques broyées ensuite à une autolyse ;
(iii) tamiser le produit intermédiaire ainsi obtenu et récupérer le filtrat de tamisage ;
(iv) précipiter les enzymes contenues dans le filtrat de tamisage ;
(v) filtrer le mélange et récupérer le gâteau de filtrage ;
(vi) moudre ensuite le gâteau de filtrage ;
(vii) le soumettre, suite à la mouture, à un séchage sous vide, et
(viii) le moudre de nouveau, pour ainsi obtenir de la pancréatine,
**caractérisé en ce que**
le gâteau de filtration tel qu'obtenu à l'étape (v), est soumis audit traitement haute pression.

5. Procédé selon l'une des revendications précédentes, la durée d'action dudit traitement haute pression étant comprise entre 1 et 60 min.

6. Procédé selon l'une des revendications précédentes, ledit traitement haute pression étant mis en oeuvre à des températures comprises entre - 20 °C et 30 °C.

7. Procédé selon l'une des revendications précédentes, ledit traitement haute pression étant mis en oeuvre à des températures comprises entre - 10 °C et 20 °C.

8. Procédé selon l'une des revendications précédentes, **caractérisé en ce que**
l'activité biologique de l'extrait biologique contenant des matières solides correspond, après ledit traitement haute pression, à au moins 90 % de l'activité biologique que l'extrait biologique contenant des matières solides présentait avant ce traitement de haute pression.

9. Procédé selon l'une des revendications précédentes, **caractérisé en ce que** le traitement haute pression de l'extrait biologique contenant des matières solides n'implique aucun ajout de substances chimiques.
